(19) **Europäisches Patentamt**
**European Patent Office**
**Office européen des brevets**

(11) **EP 1 738 682 B1**

(12) **FASCICULE DE BREVET EUROPEEN**

(45) Date de publication et mention
de la délivrance du brevet:
**13.02.2008 Bulletin 2008/07**

(51) Int Cl.:
*A61B 5/00* (2006.01)          *A61B 1/303* (2006.01)
*G01J 4/04* (2006.01)

(21) Numéro de dépôt: **05300550.0**

(22) Date de dépôt: **01.07.2005**

(54) **Système d'imagerie polarimétrique électronique pour appareil de colposcopie**

Polarimetrisch elektronisches Abbildungssystem für eine kolposkopische Vorrichtung

Electronic polarimetric imaging system for colposcopy device

(84) Etats contractants désignés:
**AT BE BG CH CY CZ DE DK EE ES FI FR GB GR HU IE IS IT LI LT LU LV MC NL PL PT RO SE SI SK TR**

(43) Date de publication de la demande:
**03.01.2007 Bulletin 2007/01**

(73) Titulaires:
• **ECOLE POLYTECHNIQUE**
  **91120 Palaiseau (FR)**
• **Institut Mutualiste Montsouris**
  **75014 Paris (FR)**
• **Assistance Publique - Hôpitaux de Paris**
  **75004 Paris (FR)**
• **Huynh, Bernard**
  **92240 Malakoff (FR)**

(72) Inventeurs:
• **De Martino, Antonello**
  **92120 Montrouge (FR)**

• **Drevillon, Bernard**
  **92140 Clamart (FR)**
• **Schwartz, Laurent**
  **75005 Paris (FR)**
• **Nazac, André**
  **75011 Paris (FR)**
• **Huynh, Bernard**
  **92240 Malakoff (FR)**

(74) Mandataire: **Michelet, Alain et al**
  **Cabinet Harlé et Phélip**
  **7, rue de Madrid**
  **75008 Paris (FR)**

(56) Documents cités:
EP-A- 1 411 333          US-A1- 2002 007 122
US-A1- 2005 090 751          US-B1- 6 175 412

**Description**

La présente invention concerne un système d'imagerie polarimétrique électronique pour appareil de colposcopie ainsi qu'un boîtier adaptateur destiné audit appareil pour réaliser un tel système. Elle a des applications dans le domaine du diagnostic médical des pathologies du col utérin.

Bien qu'il s'agisse d'une des pathologies cancéreuses les mieux maîtrisées, le cancer du col utérin induit encore une mortalité relativement importante. Or ce cancer est en général précédé d'anomalies cellulaires du col utérin dénommées dysplasies qui peuvent être détectées lors d'examens de routine. La pratique actuelle est la suivante : en cas de frottis suspect, on procède à un examen approfondi avec un appareil de colposcopie (loupe binoculaire adaptée à cet examen) au cours duquel le praticien badigeonne le col à l'acide acétique à 3%, puis au Lugol (teinture d'iode) : les zones anormales blanchissent à l'acide acétique et ne se colorent pas au Lugol. Cette technique est très sensible (très peu de faux négatifs) mais très peu spécifique (beaucoup de faux positifs) : toute anomalie, qu'elle soit bénigne ou qu'il s'agisse d'une dysplasie sévère, donne à peu près la même réponse. Avant de prendre une décision, le gynécologue pourra pratiquer des biopsies, mais le choix des endroits à biopsier reste délicat, même pour un praticien expérimenté.

En pratique, compte tenu du risque vital pour la patiente, dès qu'il a un doute le gynécologue prend la décision de pratiquer une conisation, c'est-à-dire l'ablation d'un cône d'une taille typique de 2 cm à l'avant du col. La pièce opératoire fait ensuite l'objet d'un examen histologique qui fournit le diagnostic définitif. Il apparaît ainsi a posteriori qu'un pourcentage significatif des conisations n'est pas justifié, la pièce opératoire s'avérant soit saine, soit porteuse de dysplasies légères ayant de fortes chances de régresser spontanément.

Il est donc souhaitable de disposer de moyens permettant de mieux différentier les niveaux de gravité des anomalies détectées ainsi que leur étendue afin de limiter le nombre de conisations et/ou d'en diminuer la taille. De plus, dans de nombreux pays la colposcopie est pratiquée en première intention, sans frottis préalable, ce qui peut conduire à de plus nombreux faux positifs.

On sait que les tissus sains et dysplasiques se différencient d'une part par leur architecture et d'autre part par le nombre et la taille de leurs noyaux. Ces différences peuvent donc modifier la polarisation de la lumière rétrodiffusée et des techniques d'imagerie polarimétrique peuvent donc fournir des résultats intéressants et complémentaires de ceux de la colposcopie visuelle classique, dans la mesure où l'oeil est totalement insensible à la polarisation.

Généralement, un système d'imagerie polarimétrique comporte dans son bras d'entrée un générateur d'états de polarisation, dit PSG, et dans son bras de sortie un analyseur d'états de polarisation, dit PSA. Le PSG a pour rôle de définir la séquence d'états de polarisation caractérisant la lumière incidente sur l'échantillon et le PSA analyse l'état de polarisation de la lumière réfléchie par l'échantillon, et ce, pour chacun des états de polarisation incidents définis par le PSG. Suivant le degré de caractérisation que l'on souhaite dans les mesures polarimétriques, on peut utiliser une modulation et une analyse de la polarisation plus ou moins complètes.

On va maintenant rappeler brièvement quelques principes de mesures polarimétriques applicables à l'imagerie. Pour plus de détails on pourra également se référer à la demande de brevet EP-1 411 333.

Dans le cas d'une imagerie en degré de polarisation linéaire (imagerie en DOP linéaire), le PSG est un polariseur linéaire disposé entre une source lumineuse et l'échantillon, et le PSA est un analyseur, polariseur également linéaire, disposé entre l'échantillon et une caméra. Avec un tel dispositif, on acquiert deux images, l'une avec les polariseur et analyseur parallèles ($I_{par}$) et l'autre avec les polariseur et analyseur perpendiculaires ($I_{perp}$). Typiquement, entre les deux images on tournera le polariseur de 90° dans son plan, l'analyseur restant fixe à un angle azimutal (dans son plan) α arbitraire. D'une manière alternative, on peut également utiliser un dispositif séparateur de polarisation, par exemple un cube séparateur polarisant, et deux caméras comme indiqué dans J.R. Roman, K.Lee, S.A. Prahl, S.L. Jacques, « Design, testing and clinical studies of a handheld polarized light camera » Journal of Biomedical Optics 9, 1305 (2004). A partir des images obtenues, on calcule l'image en degré de polarisation, dite DOP (« Degree Of Polarisation »), définie par

$$I_{DOP} = \frac{I_{par} - I_{perp}}{I_{par} + I_{perp}}$$

Pour des échantillons quelconques $I_{DOP}$ est théoriquement compris entre -1 et +1. En pratique, pour des échantillons de tissus biologiques, a priori dépolarisants et isotropes, $I_{DOP}$ est compris entre 0, pour un échantillon totalement dépolarisant, et 1 lorsque la polarisation incidente est totalement conservée.

Dans le cas d'une imagerie de degré de polarisation circulaire, variante de l'imagerie en degré de polarisation linéaire et dans laquelle on met en oeuvre des moyens de polarisation circulaire pour le PSG et le PSA, on considère le DOP circulaire, défini par :

$$I_{DOP,circ} = \frac{I_{gg} - I_{gd}}{I_{gg} + I_{gd}}$$

où $I_{gg}$ et $I_{gd}$ désignent respectivement des images acquises en éclairant l'échantillon en polarisation circulaire gauche et en détectant en polarisation circulaire gauche ou droite.

Dans le cas d'une imagerie de Mueller qui permet de caractériser complètement tout type d'échantillon, et en particulier des échantillons fortement dépolarisants comme les tissus biologiques optiquement épais, le PSG doit produire un ensemble complet d'états de polarisation, soit au moins quatre états dont les vecteurs de Stokes **S**i sont linéairement indépendants. Ces vecteurs de Stokes sont les vecteurs colonne de la matrice de modulation **W** caractérisant le PSG. La réflexion sur l'échantillon transforme chacun de ces vecteurs de Stokes **S**i en **M** Si , où **M** est la matrice de Mueller de l'échantillon. Le PSA détermine ensuite ces vecteurs de Stokes émergents en les projetant sur ses quatre vecteurs de Stokes caractéristiques, qui doivent être linéairement indépendants, comme les états de base du PSG, et qui forment les vecteurs ligne de la matrice d'analyse **A** caractérisant le PSA. En fin de compte, on obtient 16 images brutes, qui peuvent s'écrire (pour chaque pixel) sous la forme d'un produit de matrices réelles 4x4

$$B = A M W$$

On extrait de ces mesures brutes la matrice de Mueller **M** de l'échantillon (qui constitue donc un autre ensemble de 16 images) par inversion des matrices **A** et **W,** après détermination de ces matrices par une procédure d'étalonnage connue (on peut éventuellement consulter le document EP-1 411 333 par exemple) de l'instrument. On montre que le critère le plus pertinent pour optimiser la conception d'un polarimètre de Mueller consiste à maximiser le conditionnement des matrices **A** et **W,** c'est-à-dire le rapport de la plus petite à la plus grande de leurs valeurs singulières. On peut consulter à ce sujet les articles de Compain, B. Drévillon, dans Review of Scientific Instruments 69, 1574 (1998) ou de J.S. Tyo, dans Optics Letters 25, 1198 (2000). Quel que soit le type de polarimètre, ce rapport est limité à 0.577.

L'imagerie de Mueller est donc nettement plus compliquée à mettre en oeuvre que l'imagerie en DOP, car elle nécessite de prendre seize images au lieu de deux et l'étalonnage de l'instrument est également plus difficile. En revanche, une fois l'image de Mueller acquise, on peut très facilement en déduire par un calcul simple toutes les images qu'on aurait pu acquérir en DOP linéaire, à tous les angles azimutaux $\alpha$, ou encore l'image en DOP circulaire. De plus, contrairement au cas de l'imagerie en DOP directe, on peut ainsi afficher pour chaque pixel de l'image le DOP à l'angle $\alpha$ optimisant pour ce pixel le contraste recherché, et cet angle peut être différent d'un pixel à l'autre, par exemple à cause d'une différence d'angle d'incidence d'un endroit à l'autre de l'échantillon analysé.

Enfin, des traitements autres que le simple calcul de DOP peuvent être effectués à partir de la matrice de Mueller, comme par exemple la décomposition polaire de S. Lu and R. A. Chipman décrite dans "Interpretation of Mueller matrices based on polar decomposition", J. Opt. Soc. Am. A. 13 (5), 1106, 1996, et peuvent permettre de mieux restituer des contrastes liés à la nature du tissu lui-même plutôt qu'aux conditions d'observation. L'imagerie de Mueller permet donc une optimisation du contraste que ne permet pas l'imagerie DOP.

L'invention propose donc des moyens permettant de mettre en oeuvre une imagerie polarimétrique en colposcopie. Ces moyens peuvent être facilement couplés à un appareil de colposcopie préexistant notamment afin de réduire le coût de mise en oeuvre, tout en permettant également une utilisation classique dudit appareil. A cette fin une partie des moyens peut être facilement insérée et escamotée à la fois physiquement (pour les moyens physiques) et fonctionnellement (pour les moyens fonctionnels, notamment calculs, affichages...), de manière à permettre au praticien d'examiner chaque patiente soit en colposcopie traditionnelle (notamment en image binoculaire à haute définition ou sur écran), soit en colposcopie polarimétrique. De plus, l'invention est applicable à divers degrés de caractérisation polarimétrique du col utérin observé. C'est ainsi que dans sa version la plus simple à mettre en oeuvre mais aussi la moins complète du point de vue de la caractérisation, il est possible d'effectuer des mesures avec imagerie seulement en degré de polarisation, linéaire ou circulaire. Dans une version plus complexe à mettre en oeuvre, mais qui permet de caractériser complètement le comportement polarimétrique des éléments du col, on effectuera des mesures avec imagerie de Mueller. Dans ce dernier cas, on comprend que selon l'adage « qui peut le plus, peut le moins », étant donné la caractérisation complète possible, on puisse finalement ne considérer qu'une partie des résultats pour se limiter à des degrés de polarisation linéaire ou circulaire ou d'autres résultats partiels.

Ainsi, l'invention concerne un système d'imagerie polarimétrique électronique pour appareil de colposcopie destiné à l'observation in vivo d'un col utérin, l'appareil de colposcopie comportant une source de lumière pour éclairage du col à observer et des moyens d'observation au moins visuels d'une image dudit col, le trajet optique de l'éclairage vers le col et le trajet optique de l'image en retour du col étant séparés l'un de l'autre sur au moins une partie de leurs trajets.

Selon l'invention, le système comporte un boîtier adaptateur polarimétrique amovible dans la partie séparée des trajets optiques de l'éclairage et de l'image, ledit boîtier adaptateur polarimétrique comportant un générateur d'états de polarisation (PSG) sur le trajet optique de l'éclairage et un analyseur de polarisation (PSA) sur le trajet optique de l'image, le générateur d'états de polarisation (PSG) et l'analyseur de polarisation (PSA) étant commandés.

On comprend que l'appareil de colposcopie qui comporte des moyens d'observation au moins visuels permet au moins à l'utilisateur une observation directe visuelle. Dans le cas où seulement des moyens d'observation visuels sont disponibles, il sera préférable d'adapter audit appareil un capteur électronique d'images et au cas où ledit appareil dispose déjà d'un capteur d'image (caméra notamment) on pourra, si possible, utiliser ledit capteur pour l'imagerie. On comprend également que des moyens d'acquisition d'images provenant du capteur, de calcul/traitement des images et de visualisation des résultats sous forme d'un équipement informatique sont également mis en oeuvre. De même, l'équipement informatique permet la commande électronique des différents éléments pouvant être commandés électroniquement du système et notamment ceux du boîtier adaptateur. L'équipement informatique peut également, dans certaines versions, avoir à agir sur le capteur d'image et/ou un éventuel organe qui y est associé (filtre optique amovible par exemple). Enfin, si le système est applicable à des mesures in vivo, on comprend qu'il peut également l'être pour des mesures ex-vivo, par exemple sur des pièces opératoires.

Dans le cadre de l'invention, le terme boîtier concerne plus généralement tout moyen de support ou de maintien des PSG et PSA dans leurs relations fonctionnelles entre-eux et avec l'appareil de colposcopie en position active/fonctionnelle. Ainsi le terme boîtier peut concerner aussi bien une simple plaque support sur lesquels sont disposés les éléments, qu'une boite fermée (avec évidemment des ouvertures matérielles et fonctionnelles pour passage des trajets optiques) dans laquelle sont placés les éléments. La mise en oeuvre d'une boite présente l'avantage de pouvoir protéger de l'environnement certaines parties des éléments mis en oeuvre dans le boîtier adaptateur.

Dans divers modes de mise en oeuvre de l'invention, les moyens suivants pouvant être utilisés seuls ou selon toutes les combinaisons techniquement possibles, sont employés :

- le générateur d'états de polarisation (PSG) et l'analyseur de polarisation (PSA) sont commandés électroniquement, (mécano-électroniquement dans le cas où un moteur électrique est mis en oeuvre)
- le générateur d'états de polarisation (PSG) et l'analyseur de polarisation (PSA) sont commandés mécaniquement, (la commande peut être manuelle)
- l'appareil de colposcopie comportant une face avant en relation avec le col utérin avec au moins une pupille de sortie d'éclairage et au moins une pupille d'entrée d'image, le boîtier adaptateur polarimétrique amovible est disposé en position fonctionnelle sur ladite face avant de l'appareil,
- le boîtier adaptateur polarimétrique est amovible par translation linéaire le long de ladite face avant de l'appareil,
- le boîtier adaptateur polarimétrique est amovible par basculement avec rotation autour d'un axe parallèle à ladite face avant de l'appareil,
- le boîtier adaptateur polarimétrique est amovible par basculement avec rotation autour d'un axe perpendiculaire à ladite face avant de l'appareil,
- l'appareil de colposcopie comportant une première partie interchangeable en relation avec le col utérin et une seconde partie comportant la source de lumière et les moyens d'observation au moins visuels, le boîtier adaptateur polarimétrique amovible est disposé entre la première partie et la seconde partie de l'appareil, la première partie pouvant indifféremment se fixer sur le boîtier adaptateur polarimétrique ou sur la seconde partie,
- l'appareil comporte en outre un capteur électronique d'images,
- le boîtier adaptateur comporte en outre un capteur électronique d'images sur le trajet optique d'image, (l'image étant interceptée par le capteur, dans le cas d'un appareil monoculaire les moyens visuels de l'appareil ne sont pas utilisables par l'opérateur, dans le cas d'un appareil binoculaire, seul un des oculaires reste utilisable par l'opérateur, dans tous les cas l'utilisateur pourra se référer à un écran de visualisation sur lequel l'image provenant du capteur est affichée directement ou après calculs),
- au moins un filtre optique est disposé en amont du capteur électronique d'images,
- le filtre optique est sans effet sur l'observation visuelle,
- le filtre optique est amovible manuellement,
- le filtre optique est amovible par une commande électronique,
- le filtre optique en position active est sélectionné parmi un ensemble de filtres sélectionnables,
- le filtre optique est un passe bande,
- le passe bande est centré approximativement sur une des longueurs d'ondes suivantes : 450nm, 550nm, 650nm,
- la source de lumière pour éclairage est large bande,
- la source de lumière pour éclairage est poly-chromatique,
- la source de lumière pour éclairage est monochromatique,
- la source de lumière pour éclairage est monochromatique de longueur d'onde sélectionnable,
- la source de lumière pour éclairage est monochromatique de longueur d'onde sélectionnable avec un pic d'émission centré approximativement sur une des longueurs d'ondes suivantes : 450nm, 550nm, 650nm,

- le boîtier adaptateur comporte en outre en aval de l'analyseur de polarisation (PSA) une lame semi-réfléchissante inclinée sur le trajet optique d'image et un capteur électronique d'images, la lame semi-réfléchissante permettant de prélever une partie du signal lumineux sur le trajet optique d'image pour le renvoyer sur le capteur, (outre que l'image est effectivement envoyée dans les moyens d'observation visuels, l'image est également envoyée sur le capteur et l'utilisateur peut utiliser donc les moyens d'observation visuels que l'appareil soit monoculaire ou binoculaire)

- les moyens d'observation sont monoculaires à un seul trajet optique d'image,

- les moyens d'observation sont binoculaires à deux trajets optiques d'image et l'analyseur de polarisation (PSA) est disposé sur l'un des deux trajets optiques d'image,

- les moyens d'observation sont binoculaires à deux trajets optiques d'image et l'analyseur de polarisation (PSA) est disposé sur le trajet optique d'image destiné au capteur électronique d'images,

- dans le cas où le système peut permettre une ellipsométrie (mesures polarimétriques) complète selon les principes de Mueller, le générateur d'états de polarisation (PSG) ou l'analyseur de polarisation (PSA) est un dispositif choisi parmi :

    - un dispositif comportant un polariseur linéaire et deux modulateurs à cristaux liquides nématiques commandés électroniquement dont les axes de référence sont placés à des azimuts déterminés $\theta_{n1}$ et $\theta_{n2}$ respectivement par rapport à l'axe passant du polarisateur linéaire,

    - un dispositif comportant un polariseur linéaire et deux modulateurs à cristaux liquides ferroélectriques commandés électroniquement dont les axes de référence sont placés à des azimuts déterminés $\theta_{f1}$ et $\theta_{f2}$ respectivement par rapport à l'axe passant du polarisateur linéaire,

    - un dispositif comportant un polariseur linéaire et deux modulateurs à cristaux liquides ferroélectriques commandés électroniquement dont les axes de référence sont placés à des azimuts déterminés $\theta'_{f1}$ et $\theta'_{f2}$ respectivement par rapport à l'axe passant du polarisateur linéaire, une lame à retard fixe d'orientation déterminée $\theta'_Q$ étant interposée entre les deux modulateurs, (le générateur d'états de polarisation (PSG) ou l'analyseur de polarisation (PSA) peuvent être de même type ou de types différents)

- le système comporte des moyens tels que:

    - dans le cas d'un dispositif comportant un polariseur linéaire et deux modulateurs à cristaux liquides nématiques, les azimuts ont pour valeurs approximatives $\theta_{n1}=\varepsilon 27,4°+q90°$ +/-10° et $\theta_{n2}=\varepsilon 72,4°+r90°$ +/-10° avec $\varepsilon=+/-1$ et $q, r \in N$ , (q et r sont des entiers non nécessairement égaux)

    - dans le cas d'un dispositif comportant un polariseur linéaire et deux modulateurs à cristaux liquides ferroélectriques, les azimuts ont pour valeurs approximatives $\theta_{f1}=70°+/-5°$ et $\theta_{f2}=165°+/-5°$,

    - dans le cas d'un dispositif comportant un polariseur linéaire, deux modulateurs à cristaux liquides ferroélectriques et une lame à retard fixe, les azimuts ont pour valeurs approximatives $\theta'_{f1}=-10°+/-5°$ et $\theta'_{f2}=71°+/-5°$ et $\theta'_Q=-5°+/-2°$,

- dans le cas où le système peut permettre seulement une imagerie en DOP linéaire, le générateur d'états de polarisation (PSG) est un polariseur linéaire et l'analyseur de polarisation (PSA) comporte un modulateur à cristaux liquides ferroélectriques commandé électroniquement et un polariseur linéaire à angle de polarisation commandé électroniquement,

- dans le cas où le système peut permettre seulement une imagerie en DOP linéaire à réglage d'azimut $\alpha$, le générateur d'états de polarisation (PSG) est un polariseur linéaire et l'analyseur de polarisation (PSA) comporte un modulateur à cristaux liquides ferroélectriques commandé électroniquement, un polariseur linéaire et une lame quart d'onde solidaire dudit polariseur linéaire, la lame quart d'onde solidaire dudit polariseur linéaire ayant un axe de référence à 45° de celui du polariseur linéaire, et le générateur d'états de polarisation (PSG) et l'analyseur de polarisation (PSA) sont montés rotatifs et commandés électroniquement d'une manière synchrone en rotation d'angle $\alpha$, (afin de conserver une même relation des axes de référence des éléments formant le générateur d'états de polarisation (PSG) et l'analyseur de polarisation (PSA) entre eux et au cours de la rotation d'angle $\alpha$)

- la lame quart d'onde solidaire dudit polariseur linéaire de l'analyseur de polarisation (PSA) est une lame en mica ou une feuille plastique, notamment du type de celles proposées par la société American Polarizers Inc. (API) et fabriquées par la société 3M,

- le polariseur dichroïque est également une feuille plastique du type de celles proposées par la société American Polarizers Inc. (API) sous les références HNxx,

- le polariseur linéaire et la lame quart d'onde sont directement assemblés en un polariseur circulaire, (à titre d'exemples : les polariseurs HNCP de la société API, ou encore les nombreux polariseurs circulaires proposés par les fabricants d'accessoires photo, moyennant une vérifications de l'extinction du polariseur linéaire intégré à ces filtres, la transmission résiduelle devant être typiquement de l'ordre du pourcent à la longueur d'onde de travail)

- la commande synchrone électronique comporte un moteur dont le rotor engraine d'une part une première couronne dentée comportant intérieurement le générateur d'états de polarisation (PSG) et d'autre part une seconde couronne dentée comportant intérieurement l'analyseur de polarisation (PSA),
- dans le cas où le système peut permettre seulement une ellipsométrie circulaire, le générateur d'états de polarisation (PSG) est un polariseur circulaire et l'analyseur de polarisation (PSA) comporte un modulateur à cristaux liquides ferroélectriques commandé électroniquement, un polariseur linéaire et une lame quart d'onde solidaire dudit polariseur linéaire.

L'invention concerne également un boîtier adaptateur pour appareil de colposcopie destiné à l'observation in vivo d'un col utérin permettant de réaliser un système d'imagerie polarimétrique électronique, l'appareil de colposcopie comportant une source de lumière pour éclairage du col à observer et des moyens d'observation au moins visuels d'une image dudit col, le trajet optique de l'éclairage vers le col et le trajet optique de l'image en retour du col étant séparés l'un de l'autre sur au moins une partie de leurs trajets, dans laquelle invention, le boîtier adaptateur polarimétrique est amovible et destiné à être disposé dans la partie séparée des trajets optiques de l'éclairage et de l'image, ledit boîtier adaptateur polarimétrique comportant un générateur d'états de polarisation (PSG) sur le trajet optique de l'éclairage et un analyseur de polarisation (PSA) sur le trajet optique de l'image, le générateur d'états de polarisation (PSG) et l'analyseur de polarisation (PSA) étant commandés.

En outre, le boîtier adaptateur de l'invention peut également comporter une ou plusieurs des caractéristiques précédemment listées le concernant.

L'invention permet ainsi l'acquisition des images en polarisations parallèles et croisées d'une manière automatisée sous commande électronique, sans que le praticien n'ai à intervenir directement sur les moyens optiques mis en oeuvre. La commande électronique permet également de bénéficier d'une vitesse d'acquisition des images en polarisations parallèles croisées importante ce qui limite les risques d'artefacts dus aux possibles mouvements de la patiente entre deux acquisitions. De même, dans le cas d'un système d'acquisition directe d'imagerie DOP linéaire, l'azimut $\alpha$ de la polarisation commune de la lumière incidente et détectée en $I_{par}$ peut être ajusté facilement pour optimiser le contraste contrairement aux dispositifs traditionnels. Enfin, la disposition des moyens mis en oeuvre permet d'éviter les artefacts liés aux optiques internes de l'appareil de colposcopie, notamment dus aux séparatrices.

La présente invention va maintenant être exemplifiée sans pour autant en être limitée avec la description qui suit en relation avec les figures suivantes:

la Figure 1 qui représente schématiquement la structure d'un PSG ou d'un PSA à cristaux liquides pour imagerie de Mueller,
la Figure 2 qui représente schématiquement en perspective un boîtier adaptateur pour imagerie linéaire à azimut réglable, la Figure 3 qui représente schématiquement en vue de face un boîtier adaptateur sur la face avant d'un appareil de colposcopie typique,
la Figure 4 qui représente schématiquement en vue latérale en coupe un boîtier adaptateur positionné sur la face avant d'un appareil de colposcopie typique,
la Figure 5 qui représente en vue de face un exemple de réalisation d'un boîtier adaptateur pour imagerie linéaire à azimut réglable du type de celui de la Figure 2, et
la Figure 6 qui représente une vue en éclaté en perspective latérale du boîtier adaptateur pour imagerie linéaire à azimut réglable de la Figure 5.

Le système de l'invention permet d'utiliser un appareil de colposcopie pour effectuer des caractérisations polarimétriques de cols utérins tout en laissant la possibilité à l'utilisateur de pouvoir utiliser son appareil de colposcopie d'une manière traditionnelle. Pour cela un boîtier adaptateur amovible est mis en oeuvre ainsi que des moyens d'imagerie avec capteur d'images (dans un mode évolué). Le capteur d'image peut être disponible dans l'appareil de colposcopie et, de préférence, on l'utilisera ou, sinon, on en installera un dans l'appareil de colposcopie. Dans ce dernier cas, le fabriquant peut avoir prévu en option une telle installation de capteur d'image ou, sinon, on pourra disposer le capteur d'image d'une manière amovible en relation avec un/l'oculaire de l'appareil de colposcopie.

Ainsi, des moyens d'imagerie polarimétrique sont combinés avec un appareil de colposcopie. La modulation (PSG) et l'analyse (PSA) de la polarisation sont réalisées par des systèmes optiques utilisant des polariseurs et des cristaux liquides placés à l'avant de l'appareil de colposcopie. Ces cristaux liquides sont commandés par un équipement informatique qui gère également l'acquisition par un capteur d'images, une caméra CCD en l'espèce et placée à l'arrière de l'appareil de colposcopie et, de préférence, équipée d'un filtre sélectionnant la longueur d'onde la plus appropriée qui est typiquement dans le vert. Les PSG et PSA à cristaux liquides mis en oeuvre permettent de moduler et analyser la polarisation avec rapidité et sont compacts car l'encombrement des PSG et PSA est très réduit, aussi bien transversalement que longitudinalement.

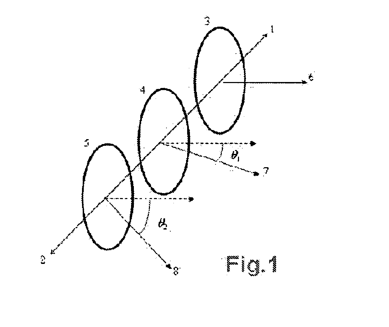

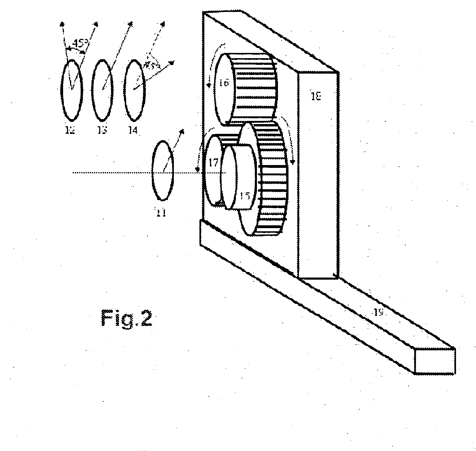

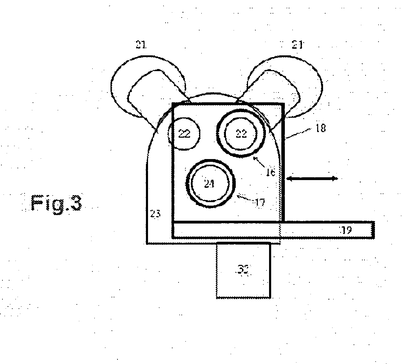

On va maintenant décrire les PSG et PSA utilisables dans le boîtier adaptateur pour une imagerie de Mueller. D'une manière générale, les PSG et PSA comportent un polariseur linéaire, qui, pour l'application envisagée, est placé contre la face avant de l'appareil de colposcopie, et deux modulateurs à cristaux liquides dont les axes de référence sont placés à des azimuts $\theta_1$ et $\theta_2$ par rapport à l'axe passant du polariseur linéaire comme représenté Figure 1. Ainsi dans un PSG ou un PSA, sur un trajet optique 1-2, 1 étant vers la face avant de l'appareil de colposcopie et 2 étant vers le col utérin à observer, on trouve un polariseur linéaire 3 avec une direction 6 de l'axe passant du polariseur linéaire et deux modulateurs à cristaux liquides 4 et 5 commandés électroniquement et qui ont des directions des axes de référence 7, 8 des cristaux liquides dans le plan des modulateurs. Ce type de PSG et PSA permet d'obtenir toute l'information polarimétrique sans qu'il soit nécessaire de faire bouger quelque élément optique que ce soit (sauf le boîtier adaptateur dans son ensemble pour l'escamoter) et donc les PSG et PSA pourront être fixés, en l'espèce sur un support 18 (Figures 2 ou 3) sans possibilité de rotation individuelle.

Pour des performances optimales, l'orientation concrète des PSG et PSA est typiquement imposée par le dichroïsme de l'appareil de colposcopie. Par exemple, lors d'essais, il a été observé que l'intensité transmise à la caméra CCD est beaucoup plus faible, plus d'un facteur 10, en polarisation verticale qu'en polarisation horizontale, ce que l'on peut expliquer par présence de séparatrices standard dans l'appareil de colposcopie qui a été utilisé pour les essais. On pourra donc être amené à régler les orientations des PSG et PSA en fonction de l'appareil de colposcopie utilisé dans le système de l'invention. Ce réglage, effectué une fois pour toutes, n'est cependant pas critique

D'autre part, il peut être utile d'effectuer une procédure d'étalonnage qui est connue en soit (par exemple dans la demande de brevet EP-1,411,333) pour définir l'origine des angles azimutaux indépendamment de l'orientation effective des PSG et PSA dans le boîtier adaptateur: cette origine ne dépend en fait que de l'orientation des éléments optiques additionnels (polariseurs, lames retard) utilisés pendant la procédure d'étalonnage.

On vient donc de décrire d'une manière schématique les PSG et PSA utilisables pour une imagerie de Mueller. Concrètement, plusieurs modalités de réalisation des PSG et PSA sont utilisables. Ainsi, outre les polariseurs linéaires, les PSG et PSA pourront comporter soit deux cristaux liquides nématiques, soit deux cristaux liquides ferroélectriques, soit deux cristaux liquides ferroélectriques et une lame retard fixe entre les deux.

Dans le cas de deux cristaux liquides nématiques, on a une équivalence avec des lames retard d'orientation fixe et de retard variable, commandé électroniquement. Pour ce type de modulateur, l'axe de référence dans le plan correspond à l'axe extraordinaire, d'indice variable. Les états de polarisation Si sont générés séquentiellement, en imposant des retards $(\delta_1, \delta_2)$ aux deux cristaux liquides dans une séquence de la forme

$$(\delta_1, \delta_2) = (\Delta_1, \Delta_1), (\Delta_2, \Delta_1), (\Delta_1, \Delta_2), (\Delta_2, \Delta_2),$$

Cette configuration permet de maintenir le conditionnement des matrices A et W près de la valeur optimale 0,577 quelle que soit la longueur d'onde (du moins dans le visible et le proche infrarouge) pour les valeurs suivantes des paramètres:

$$\theta_1 = \varepsilon\, 27{,}4° + q90° \qquad \text{et} \qquad \theta_2 = \varepsilon\, 72{,}4° + r90° ,$$

$$\Delta_1 = 315° + p90° \qquad \text{et} \qquad \Delta_2 = 135° + p90°$$

avec $\theta_1$ et $\theta_2$ à +/-10° et où $\varepsilon = \pm 1$ a la même valeur dans les deux équations, alors que q et r sont des nombres entiers quelconques (pas nécessairement égaux).

Dans le cas de deux cristaux liquides ferroélectriques on a une équivalence avec des lames retard dont le retard est fixe et que la commande électronique fait basculer entre deux orientations à 45° l'une de l'autre, sans changer la valeur du retard. Les quatre vecteurs de Stokes Si du PSG et du PSA sont donc obtenus, en partant d'un état initial (pour le PSG) ou final (pour le PSA) polarisé linéairement, par traversée des deux lames ferroélectriques dont les orientations des axes lents prennent successivement les valeurs :

$$(\theta_1, \theta_2),\ (\theta_1+45°, \theta_2),\ (\theta_1, \theta_2+45°),\ (\theta_1+45°, \theta_2+45°)$$

Par rapport aux nématiques, les ferroélectriques présentent l'avantage de commuter beaucoup plus rapidement, mais ils ne permettent pas de maintenir un conditionnement optimum sur une large gamme spectrale aussi large. Néanmoins, avec des modulateurs commerciaux, spécifiés demi-onde et quart d'onde à 510 nm, on obtient un conditionnement acceptable (supérieur à 0,4) sur toute la gamme spectrale entre 500 et 650 nm, pour les orientations suivantes :

$$\theta_1 = 70° \pm 5°, \quad \theta_2 = 165° \pm 5°$$

Dans le cas de deux cristaux liquides ferroélectriques et une lame retard fixe entre les deux. Les mêmes ferroélectriques peuvent être utilisés dans une gamme spectrale sensiblement plus large (de 450 à 750 nm) si on intercale entre les deux une lame retard fixe, en quartz, spécifiée quart d'onde à 633 nm. Les orientations de ces trois éléments sont alors les suivantes :

$$\theta_1 = -10° \pm 5°, \quad \theta_Q = -5° \pm 2° \quad \theta_2 = 71° \pm 5°$$

On va maintenant décrire le système dans le cas où l'on limite la caractérisation en DOP linéaire. Pour mettre en oeuvre une imagerie en DOP linéaire à azimut réglable le PSG peut se réduire à un polariseur linéaire et le PSA à un ferroélectrique et un analyseur linéaire. Les axes passants du polariseur et de l'analyseur linéaires étant parallèles, il suffit de commuter le ferroélectrique pour acquérir séquentiellement, et en un temps très court, les images en polarisations parallèles et croisées. Le système correspondant est illustré sur les Figures 2 à 6 et permet d'acquérir des images en DOP linéaire, tout en réglant facilement l'azimut α de la polarisation incidente sans désaligner les polariseur et analyseur linéaires. A cette fin, on insère les PSG et PSA dans des couronnes dentées entraînées par une même roue dentée de commande. De plus, pour éviter des variations trop importantes de l'intensité détectée avec l'azimut, on place derrière l'analyseur linéaire une lame quart d'onde solidaire de cet analyseur et dont les axes sont à 45° de celui-ci, pour convertir la polarisation linéaire après l'analyseur en polarisation circulaire, en principe indépendante de l'azimut α: l'intensité finalement détectée doit donc être indépendante de α, même pour un appareil de colposcopie présentant un dichroïsme linéaire important. L'assemblage polariseur linéaire-lame quart d'onde peut être avantageusement remplacée par un polariseur circulaire associant ces deux éléments dans une feuille plastique d'aspect analogue aux classiques polariseurs dichroïques de type « Polaroïd® », à condition toutefois que le taux d'extinction du polariseur linéaire inclus dans ce polariseur circulaire soit suffisant (de l'ordre de 100 ou plus). Notons que d'une manière alternative, rien n'interdit de placer le ferroélectrique dans le PSG, après le polariseur linéaire, et de réduire le PSA au seul polariseur circulaire.

La Figure 2 permet de visualiser le boîtier adaptateur qui est dans ce mode de réalisation un simple support 18 monté coulissant sur un rail 19 afin de permettre son escamotage de la face avant d'un appareil de colposcopie lorsque ce dernier doit être utilisé d'une manière classique. Le PSG 17 est fixé dans une couronne dentée et on a représenté sur la partie gauche de la figure sa fonction sous forme d'un polariseur linéaire 11 avec la flèche indiquant la direction de l'axe passant. Le PSA 16 est fixé dans une couronne dentée et on a représenté sur la partie gauche de la figure sa fonction sous forme de la combinaison d'un cristal liquide ferroélectrique 12 (les flèches indiquent les deux directions possibles de l'axe lent), d'un analyseur linéaire 13 dont l'axe passant (flèche) est parallèle à celui du polariseur 11 du PSG et d'une lame quart d'onde 14 à 45° de l'axe des polariseur et analyseur. Une roue dentée 15 engraine les couronnes portant les PSG et PSA. La roue dentée peut être commandée manuellement afin de rechercher le meilleur angle α d'azimut et/ou, dans une variante, commandé électroniquement (motorisation). La face avant de l'appareil de colposcopie (non représenté sur la Figure 2) est à droite de la figure et la direction d'observation du col utérin à gauche.

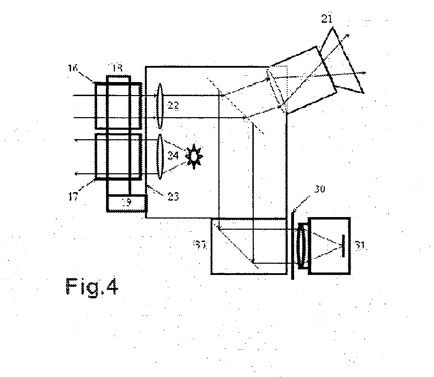

Les Figures 3 et 4 permettent de visualiser les relations fonctionnelles et structurelles entre le boîtier adaptateur et l'appareil de colposcopie dans le système de l'invention. Sur ces Figures, le boîtier adaptateur, comportant une plaque support 18 en l'espèce, est représenté en traits épais alors que l'appareil de colposcopie est représenté en traits fins. On a également représenté en traits épais le capteur d'images mais on rappelle que celui-ci peut aussi bien faire partie de l'appareil de colposcopie (accessoire fourni) ou qu'il peut être rajouté pour les besoins de l'imagerie DOP.

L'appareil de colposcopie est ici du type binoculaire avec deux oculaires ou oeilletons 21 vers l'arrière pour l'observation visuelle. Sur la face avant 23 de l'appareil de colposcopie on trouve deux pupilles d'entrée 22 du système d'imagerie binoculaire et une pupille de sortie 24 du système d'éclairage. L'appareil de colposcopie comporte une sortie pour prise d'images sur laquelle on a un filtre optique 30 et une caméra CCD 31. La prise d'image se fait sur une des deux images du système binoculaire grâce à une lame représentée en traits discontinus Figure 4. On retrouve sur la face avant de l'appareil de colposcopie dans le boîtier adaptateur amovible l'analyseur d'états de polarisation (PSA) 16 et le générateur d'états de polarisation (PSG) 17 qui sont sur une plaque support 18 commune aux PSG et PSA et pouvant coulisser (comme indiqué par la flèche en trait épais Figure 3) sur le rail 19 et permettant ainsi son escamotage.

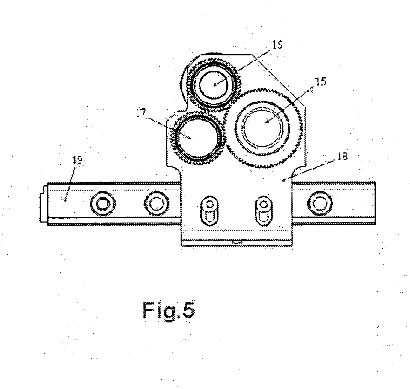

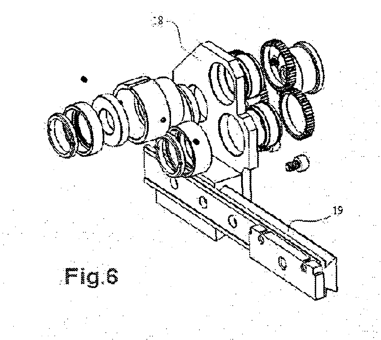

La Figure 5 est un exemple de réalisation concret du boîtier adaptateur du type schématisé sur les Figures 3 et 4 en vue de face et la Figure 6 une vue perspective en éclaté.

Dans une variante, il est possible d'effectuer une imagerie en DOP circulaire qui présente l'avantage de supprimer la nécessité de faire tourner les PSG et PSA pour optimiser le contraste, mais ce dernier sera a priori plus faible qu'en DOP linéaire pour une orientation optimisée de l'angle azimutal $\alpha$. Pour mettre en oeuvre cette variante, il suffit que le PSG soit constitué d'un polariseur circulaire et le PSA d'un analyseur circulaire alternativement gauche et droit, obtenu en plaçant un cristal ferroélectrique demi-onde entre le polariseur linéaire et la lame quart d'onde constituant l'analyseur circulaire. D'une manière alternative, on peut tout aussi bien placer ces trois éléments dans le PSG et limiter le PSA à un analyseur circulaire.

Des expériences ont été réalisées en imagerie DOP linéaire sur des pièces opératoires ex-vivo (juste après exérèse) de cols utérins, avant l'envoi chez l'anatomopathologiste. Les contrastes visibles sur les images ont été comparés avec les indications du rapport d'anatomopathologie. Plusieurs longueurs d'onde ont été utilisées et on a pu faire apparaître en imagerie DOP des dysplasies sévères qui étaient invisible sur les images non polarisées. On a pu constater sur les images que le contraste varie fortement en fonction de l'angle azimutal $\alpha$ définissant l'orientation commune des polariseur et analyseur pour l'acquisition de l'image ($I_{par}$). Cette dépendance est probablement liée à l'incidence élevée sous laquelle on observe le tissu du col utérin dans la zone centrale de l'organe (entrée de l'endocol). L'imagerie en DOP circulaire permet a priori de s'affranchir de la dépendance du contraste en fonction de l'azimut $\alpha$ qu'on observe en DOP linéaire mais au prix d'un contraste un peu plus faible qu'en DOP linéaire optimisé en azimut.

## Revendications

1. Boîtier adaptateur polarimétrique (15) pour appareil de colposcopie destiné à l'observation in vivo d'un col utérin, l'appareil de colposcopie comportant une source de lumière pour éclairage du col à observer et des moyens d'observation au moins visuels d'une image dudit col, le trajet optique de l'éclairage vers le col et le trajet optique de l'image en retour du col étant séparés l'un de l'autre sur au moins une partie de leurs trajets, **caractérisé en ce que** le boîtier adaptateur polarimétrique (18) est amovible et adapté à être disposé dans la partie séparée des trajets optiques de l'éclairage et de l'image, ledit boîtier adaptateur polarimétrique comportant un générateur d'états de polarisation (PSG) (17) sur le trajet optique de l'éclairage et un analyseur de polarisation (PSA) (16) sur le trajet optique de l'image, le générateur d'états de polarisation (PSG) (17) et l'analyseur de polarisation (PSA) (16) étant commandés.

2. Système d'imagerie polarimétrique électronique comportant un appareil de colposcopie destiné à l'observation in vivo d'un col utérin, l'appareil de colposcopie comportant une source de lumière pour éclairage du col à observer et des moyens d'observation au moins visuels d'une image dudit col, le trajet optique de l'éclairage vers le col et le trajet optique de l'image en retour du col étant séparés l'un de l'autre sur au moins une partie de leurs trajets, **caractérisé en ce qu'**il comporte en outre un boîtier adaptateur selon la revendication 1, le boîtier adaptateur polarimétrique étant amovible dans la partie séparée des trajets optiques de l'éclairage et de l'image, ledit boîtier adaptateur polarimétrique comportant un générateur d'états de polarisation (PSG) sur le trajet optique de l'éclairage et un analyseur de polarisation (PSA) sur le trajet optique de l'image, le générateur d'états de polarisation (PSG) et l'analyseur de polarisation (PSA) étant commandés.

3. Système selon la revendication 2, **caractérisé en ce que** l'appareil de colposcopie comportant une face avant en relation avec le col utérin avec au moins une pupille de sortie d'éclairage et au moins une pupille d'entrée d'image, le boîtier adaptateur polarimétrique amovible est disposé en position fonctionnelle sur ladite face avant de l'appareil.

4. Système selon la revendication 3, **caractérisé en ce que** le boîtier adaptateur polarimétrique est amovible par un moyen choisi parmi :

   - une translation linéaire le long de ladite face avant de l'appareil,
   - un basculement avec rotation autour d'un axe parallèle à ladite face avant de l'appareil,
   - une rotation autour d'un axe perpendiculaire à ladite face avant de l'appareil.

5. Système selon l'une quelconque des revendications 2 à 4, **caractérisé en ce que** l'appareil comporte en outre un capteur électronique d'images.

6. Système selon l'une quelconque des revendications 2 à 5, **caractérisé en ce que** le boîtier adaptateur comporte en outre un capteur électronique d'images sur le trajet optique d'image.

7. Système selon l'une quelconque des revendications 2 à 5, **caractérisé en ce que** le boîtier adaptateur comporte en outre en aval de l'analyseur de polarisation (PSA) une lame semi-réfléchissante inclinée sur le trajet optique d'image et un capteur électronique d'images, la lame semi-réfléchissante permettant de prélever une partie du signal lumineux sur le trajet optique d'image pour le renvoyer sur le capteur.

8. Système selon l'une quelconque des revendications 2 à 7, **caractérisé en ce que** dans le cas où le système peut permettre une ellipsométrie complète selon les principes de Mueller, le générateur d'états de polarisation (PSG) ou l'analyseur de polarisation (PSA) est un dispositif choisi parmi :

   - un dispositif comportant un polariseur linéaire et deux modulateurs à cristaux liquides nématiques commandés électroniquement dont les axes de référence sont placés à des azimuts déterminés $\theta_{n1}$ et $\theta_{n2}$ respectivement par rapport à l'axe passant du polarisateur linéaire,
   - un dispositif comportant un polariseur linéaire et deux modulateurs à cristaux liquides ferroélectriques commandés électroniquement dont les axes de référence sont placés à des azimuts déterminés $\theta_{f1}$ et $\theta_{f2}$ respectivement par rapport à l'axe passant du polarisateur linéaire,
   - un dispositif comportant un polariseur linéaire et deux modulateurs à cristaux liquides ferroélectriques commandés électroniquement dont les axes de référence sont placés à des azimuts déterminés $\theta'_{f1}$ et $\theta'_{f2}$ respectivement par rapport à l'axe passant du polarisateur linéaire, une lame à retard fixe d'orientation déterminée $\theta'_Q$ étant interposée entre les deux modulateurs.

9. Système selon la revendication 8, **caractérisé en ce que** :

   - dans le cas d'un dispositif comportant un polariseur linéaire et deux modulateurs à cristaux liquides nématiques, les azimuts ont pour valeurs approximatives $\theta_{n1}= \varepsilon 27,4°+q90°$ +/-10° et $\theta_{n2}=\varepsilon 72,4°+r90°$ +/-10° avec $\varepsilon$=+/-1 et q, r $\in$ N,
   - dans le cas d'un dispositif comportant un polariseur linéaire et deux modulateurs à cristaux liquides ferroélectriques, les azimuts ont pour valeurs approximatives $\theta_{f1}=70°$+/-5° et $\theta_{f2}=165°$+/-5°,
   - dans le cas d'un dispositif comportant un polariseur linéaire, deux modulateurs à cristaux liquides ferroélectriques et une lame à retard fixe, les azimuts ont pour valeurs approximatives $\theta'_{f1}=-10°$+/-5° et $\theta'_{f2}=71°$+/-5° et $\theta'_Q=-5°$+/-2°.

10. Système selon l'une quelconque des revendications 2 à 7, **caractérisé en ce que** dans le cas où le système peut permettre seulement une imagerie en DOP linéaire, le générateur d'états de polarisation (PSG) est un polariseur linéaire et l'analyseur de polarisation (PSA) comporte un modulateur à cristaux liquides ferroélectriques commandé électroniquement et un polariseur linéaire à angle de polarisation commandé électroniquement.

11. Système selon l'une quelconque des revendications 2 à 7, **caractérisé en ce que** dans le cas où le système peut permettre seulement une imagerie en DOP linéaire à réglage d'azimut $\alpha$, le générateur d'états de polarisation (PSG) est un polariseur linéaire et l'analyseur de polarisation (PSA) comporte un modulateur à cristaux liquides ferroélectriques commandé électroniquement, un polariseur linéaire et une lame quart d'onde solidaire dudit polariseur linéaire, la lame quart d'onde solidaire dudit polariseur linéaire ayant un axe de référence à 45° de celui du polariseur linéaire, et **en ce que** le générateur d'états de polarisation (PSG) et l'analyseur de polarisation (PSA) sont montés rotatifs et commandés électroniquement d'une manière synchrone en rotation d'angle $\alpha$.

12. Système selon la revendication 11, **caractérisé en ce que** dans le cas la commande électronique synchrone comporte un moteur dont le rotor engraine d'une part une première couronne dentée comportant intérieurement le générateur d'états de polarisation (PSG) et d'autre part une seconde couronne dentée comportant intérieurement l'analyseur de polarisation (PSA).

13. Système selon l'une quelconque des revendications 2 à 7, **caractérisé en ce que** dans le cas où le système peut permettre seulement une ellipsométrie circulaire, le générateur d'états de polarisation (PSG) est un polariseur circulaire et l'analyseur de polarisation (PSA) comporte un modulateur à cristaux liquides ferroélectriques commandé électroniquement, un polariseur linéaire et une lame quart d'onde solidaire dudit polariseur linéaire.

**Claims**

1. A polarimetric housing (18) for a colposcopy device designed for in vivo observation of a cervix enabling to provide an electronic polarimetric imaging system, wherein the colposcopy device comprises a light source for illuminating the observable cervix and at least visual means for monitoring an image of said cervix, the illumination optical path towards the cervix and the image optical path coming back from the cervix being separated from one another over at least one portion of the paths thereof,
**characterised in that** the polarimetric adapter housing (18) is removable and adapted for being arranged in the separated portion of the illumination and image optical paths, said polarimetric adapter housing including a polarisation state generator (PSG) (17) on the illumination optical path and a polarisation analyser (PSA) (16) on the image optical path, wherein the polarisation state generator (PSG) (17) and the polarisation analyser (PSA) (16) are controllable.

2. An electronic polarimetric imaging system comprising a colposcopy device designed for in vivo observation of a cervix, wherein the colposcopy device comprising a light source for illuminating the observable cervix and at least visual means for monitoring an image of said cervix, the illumination optical path towards the cervix and the image optical path coming back from the cervix being separated from one another over at least one portion of the paths thereof,
**characterised in that** it comprises moreover a polarimetric adapter housing according to claim 1, the polarimetric adapter housing being removable into the separated portion of the illumination and image optical paths, said polarimetric adapter housing including a polarisation state generator (PSG) on the illumination optical path and a polarisation analyser (PSA) on the image optical path, the polarisation state generator (PSG) and the polarisation analyser (PSA) being controllable.

3. A system according to claim 2, **characterised in that** the colposcopy device comprising a front face in relation with the cervix with at least one illumination output pupil and at least one image input pupil, the removable polarimetric adapter housing is arranged in functional position on said front face of the device.

4. A system according to claim 3, **characterised in that** the polarimetric adapter housing is removable by a means selected among:

   - a linear translation along said front face of the device,
   - a tilting-over with rotation around an axis parallel to said front face of the device,
   - a rotation around an axis perpendicular to said front face of the device.

5. A system according to any of the claims 2 to 4, **characterised in that** the device includes moreover an image electronic sensor.

6. A system according to any of the claims 2 to 5, **characterised in that** the adapter housing includes moreover an image electronic sensor on the image optical path.

7. A system according to any of the claims 2 to 5, **characterised in that** the adapter housing includes moreover downstream of the polarisation analyser (PSA) a semi-reflecting blade tilted on the image optical path and an image electronic sensor, wherein the semi-reflecting blade enables to sample a portion of the light signal on the image optical path to send it back to the sensor.

8. A system according to any of the claims to 2 to 7, **characterised in that** in case when the system may enable complete ellipsometry according to the Mueller principles, the polarisation state generator (PSG) or the polarisation analyser (PSA) is a device selected among:

   - a device including a linear polariser and two electronically controlled nematic liquid crystal modulators whereof the reference axes are situated at determined azimuths $\theta_{n1}$ and $\theta_{n2}$ respectively relative to the conducting axis of the linear polariser,
   - a device including a linear polariser and two electronically controlled ferroelectric liquid crystal modulators whereof the reference axes are situated at determined azimuths $\theta_{f1}$ and $\theta_{f2}$ respectively relative to the conducting axis of the linear polariser,
   - a device including a linear polariser and two electronically controlled ferroelectric liquid crystal modulators whereof the reference axes are situated at determined azimuths $\theta'_{f1}$ and $\theta'_{f2}$ respectively relative to the conducting axis of the linear polariser, wherein a fixed delay blade of determined orientation $\theta'_Q$ is interposed between both modulators.

9. A system according to claim 8, **characterised in that**:

   - in the case of a device including a linear polariser and two nematic liquid crystal modulators, the azimuths have as approximate values $\theta_{n1}= \varepsilon 27.4°+q90°$ +/-10° and $\theta_{n2}=\varepsilon 72.4°+r90°$ +/-10° with $\varepsilon=$+/-1 and q, r $\in$ N
   - in the case of a device including a linear polariser and two ferroelectric liquid crystal modulators, the azimuths have as approximate values $\theta_{f1}=70°$+/-5° and $\theta_{f2}=165°$+/-5°,
   - in the case of a device including a linear polariser, two ferroelectric liquid crystal modulators and a fixed delay blade, the azimuths have as approximate values $\theta'_{f1}=-10°$+/-5° and $\theta'_{f2}=71°$+/-5° and $\theta'_Q=-5°$+/-2°.

10. A system according to any of the claims 2 to 7, **characterised in that** in case when the system may enable only linear DOP imaging, the polarisation state generator (PSG) is a linear polariser and the polarisation analyser (PSA) includes an electronically controlled ferroelectric liquid crystal modulator and a linear polariser provided with an electronically controlled polarisation angle.

11. A system according to any of the claims 2 to 7, **characterised in that** in case when the system may enable only linear DOP imaging with azimuth setting $\alpha$, the polarisation state generator (PSG) is a linear polariser and the polarisation analyser (PSA) includes an electronically controlled ferroelectric liquid crystal modulator, a linear polariser and a quarter wave blade interconnected with said linear polariser, wherein the quarter wave blade interconnected with said linear polariser has a reference axis of 45° relative to that of the linear polariser, and **in that** the polarisation state generator (PSG) and the polarisation analyser (PSA) are mounted rotatably and controlled electronically synchronously and rotatably by an angle $\alpha$.

12. A system according to claim 11, **characterised in that** in the case when the synchronous electronic control includes a motor, whereof the rotor meshes on the one hand into a first toothed ring containing internally the polarisation state generator (PSG) and on the other hand a second toothed ring containing internally the polarisation analyser (PSA).

13. A system according to any of the claims 2 to 7, **characterised in that** in case when the system may enable only circular ellipsometry, the polarisation state generator (PSG) is a circular polariser and the polarisation analyser (PSA) includes an electronically controlled ferroelectric liquid crystal modulator, a linear polariser and a quarter-wave blade interconnected with said linear polariser.

**Patentansprüche**

1. Polarimetrisches Adaptorgehäuse (18) für ein Kolposkopiegerät, das zur In-vivo-Beobachtung eines Gebärmutterhalses bestimmt ist, wobei das Kolposkopiegerät eine Lichtquelle zum Ausleuchten des zu beobachtenden Halses und mindestens visuelle Beobachtungsmittel eines Bildes dieses Halses umfasst, wobei der optische Weg der Ausleuchtung zum Hals hin und der optische Weg des Bildes vom Hals zurück voneinander auf mindestens einem Teil ihrer Wege getrennt sind,
   **dadurch gekennzeichnet, dass** das polarimetrische Adaptorgehäuse (18) abnehmbar ist und derart ausgeführt ist, dass es in dem getrennten Teil des optischen Weges der Ausleuchtung und des Bildes angeordnet ist, wobei das polarimetrische Adaptorgehäuse auf dem optischen Weg der Ausleuchtung einen Generator von Polarisationszuständen (PSG) (17) und einen Polorisationsanalysator (PSA) (16) auf dem optischen Weg des Bildes umfasst, wobei der Generator von Polarisationszuständen (PSG) (17) und der Polarisationsanalysator (PSA) (16) gesteuert werden.

2. Elektronisches polarimetrisches Abbildungssystem, umfassend ein Kolposkopiegerät, das zur In-vivo-Beobachtung eines Gebärmutterhalses bestimmt ist, wobei das Kolposkopiegerät eine Lichtquelle zum Ausleuchten des zu beobachtenden Halses und mindestens visuelle Beobachtungsmittel eines Bildes dieses Halses umfasst, wobei der optische Weg der Ausleuchtung zum Hals hin und der optische Weg des Bildes vom Hals zurück voneinander auf mindestens einem Teil ihres Weges getrennt sind,
   **dadurch gekennzeichnet, dass** es ferner ein Adaptorgehäuse nach Anspruch 1 umfasst, wobei das polarimetrische Adaptorgehäuse in dem getrennten Teil der optischen Wege der Ausleuchtung und des Bildes abnehmbar ist, wobei das polorimetrische Adaptorgehäuse auf dem optischen Weg der Ausleuchtung einen Generator von Polarisationszuständen (PSG) und auf dem optischen Weg des Bildes einen Polarisationsanalysator (PSA) umfasst, wobei der Generator von Polarisationszuständen (PSG) und der Polarisationsanalysator (PSA) gesteuert werden.

3. System nach Anspruch 2, **dadurch gekennzeichnet, dass** das Kolposkopiegerät eine Vorderseite in Bezug auf den Gebärmutterhals mit mindestens einer Austrittspupille der Ausleuchtung und mindestens einer Eintrittspupille des Bildes umfasst, wobei das abnehmbare polarimetrische Adaptorgehäuse in Funktionsstellung auf der Vorderseite des Geräts angeordnet ist.

4. System nach Anspruch 3, **dadurch gekennzeichnet, dass** das polarimetrische Adaptorgehäuse durch ein Mittel abnehmbar ist, das ausgewählt ist aus:

   - einer linearen Verschiebung entlang der Vorderseite des Geräts,
   - einer Kippbewegung um eine Achse, die parallel zur Vorderseite des Geräts liegt,
   - einer Drehung um eine Achse, die senkrecht zur Vorderseite des Geräts liegt.

5. System nach einem der Ansprüche 2 bis 4, **dadurch gekennzeichnet, dass** das Gerät ferner einen elektronischen Bildsensor umfasst.

6. System nach einem der Ansprüche 2 bis 5, **dadurch gekennzeichnet, dass** das Adaptorgehäuse ferner einen elektronischen Bildsensor auf dem optischen Weg des Bildes umfasst.

7. System nach einem der Ansprüche 2 bis 5, **dadurch gekennzeichnet, dass** das Adaptorgehäuse ferner unterhalb des Polarisationsanalysators (PSA) eine halb reflektierende Klinge, die über den optischen Weg des Bildes geneigt ist, und einen elektronischen Bildsensor umfasst, wobei die halb reflektierende Klinge das Entnehmen eines Teils des Lichtsignals auf dem optischen Weg des Bildes, um es der Sonde zurückzusenden, erlaubt.

8. System nach einem der Ansprüche 2 bis 7, **dadurch gekennzeichnet, dass** im Falle, dass das System eine vollständige Ellipsometrie nach den Prinzipien von Müller erlauben kann, der Generator von Polarisationszuständen (PSG) oder der Polarisationsanalysator (PSA) eine Vorrichtung ist, die ausgewählt ist aus:

   - einer Vorrichtung, umfassend einen linearen Polarisator und zwei Modulatoren mit nematischen Flüssigkristallen, die elektronisch gesteuert werden, deren Referenzachsen unter bestimmte Azimutwinkel $\theta_{n1}$ bzw. $\theta_{n2}$ hinsichtlich der Achse am linearen Polarisator vorbei gesetzt sind,
   - einer Vorrichtung, umfassend einen linearen Polarisator und zwei Modulatoren mit ferroelektrischen Flüssigkristallen, die elektronisch gesteuert werden, deren Referenzachsen unter bestimmte Azimutwinkel $\theta_{f1}$ bzw. $\theta_{f2}$ hinsichtlich der Achse am linearen Polarisator vorbei gesetzt sind,
   - einer Vorrichtung, umfassend einen linearen Polarisator und zwei Modulatoren mit ferroelektrischen Flüssigkristallen, die elektronisch gesteuert werden, deren Referenzachsen unter bestimmte Azimutwinkel $\theta'_{f1}$ bzw. $\theta'_{f2}$ hinsichtlich der Achse am linearen Polarisator vorbei gesetzt sind, wobei eine feste Verzögerungsklinge mit bestimmter Ausrichtung $\theta'_{Q}$ zwischen die beiden Modulatoren geschoben ist.

9. System nach Anspruch 8, **dadurch gekennzeichnet, dass**:

   - im Falle, dass eine Vorrichtung einen linearen Polarisator und zwei Modulatoren mit nematischen Flüssigkristallen umfasst, die annähernden Werte der Azimutwinkel $\theta_{n1} = \varepsilon 27{,}40° + q90° +/- 10°$ und $\theta_{n2} = \varepsilon 72{,}4° + r90° +/- 10°$, wobei $\varepsilon = +/-1$ und $q, r \in N$, betragen,
   - im Falle, dass eine Vorrichtung einen linearen Polarisator und zwei Modulatoren mit ferroelektrischen Flüssigkristallen umfasst, die annähernden Werte der Azimutwinkel $\theta_{f1} = 70° +/- 5°$ und $\theta_{f2} = 165° +/- 5°$ betragen,
   - im Falle, dass eine Vorrichtung einen linearen Polarisator, zwei Modulatoren mit ferroelektrischen Flüssigkristallen und eine Klinge mit fester Verzögerung umfasst, die annähernden Werte der Azimutwinkel $\theta'_{f1} = -10° +/- 5°$ und $\theta'_{f2} = 71° +/- 5°$ und $\theta'_{Q} = -5° +/- 2°$ betragen.

10. System nach einem der Ansprüche 2 bis 7, **dadurch gekennzeichnet, dass** im Falle, dass das System lediglich eine Bildverarbeitung in linearem DOP erlauben kann, wobei der Generator von Polarisationszuständen (PSG) ein linearer Polarisator ist und der Polarisationsanalysator (PSA) einen Modulator mit ferroelektrischen Flüssigkristallen, welcher elektronisch gesteuert wird, und einen linearen Polarisator mit elektronisch gesteuertem Polarisationswinkel umfasst.

**11.** System nach einem der Ansprüche 2 bis 7, **dadurch gekennzeichnet, dass** im Falle, dass das System lediglich eine Bildverarbeitung in linearem DOP mit Spalteinstellung a erlauben kann, der Generator von Polarisationszuständen (PSG) ein linearer Polarisator ist und der Polarisationsanalysator (PSA) einen Modulator mit ferroelektrischen Flüssigkristallen, welcher elektronisch gesteuert wird, einen linearen Polarisator und eine Viertelklinge, die mit dem linearen Polarisator fest verbunden ist, umfasst, wobei die Viertelklinge, die mit dem linearen Polarisator fest verbunden ist, eine Referenzachse von 45° zu derjenigen des linearen Polarisators aufweist, und **dadurch**, dass der Generator von Polarisationszuständen (PSG) und der Polarisationsanalysator (PSA) drehbar angebracht sind und elektronisch auf synchrone Weise mit der Winkeldrehung a gesteuert werden.

**12.** System nach Anspruch 11, **dadurch gekennzeichnet, dass** im Falle, dass die synchrone elektronische Steuerung einen Motor umfasst, dessen Rotor einerseits einen ersten Zahnkranz, der im Inneren den Generator von Polarisationszuständen (PSG) umfasst, und andererseits einen zweiten Zahnkranz, der im Inneren den Polarisationsanalysator (PSA) umfasst, antreibt.

**13.** System nach einem der Ansprüche 2 bis 7, **dadurch gekennzeichnet, dass** im Falle, dass das System lediglich eine kreisförmige Ellipsometrie erlauben kann, der Generator von Polarisationszuständen (PSG) ein kreisförmiger Polarisator ist und der Polarisationsanalysator (PSA) einen Modulator mit ferroelektrischen Flüssigkristallen, der elektronisch gesteuert wird, einen linearen Polarisator und eine Viertelklinge, die mit dem linearen Polarisator fest verbunden ist, umfasst.

Fig.1

Fig.2

Fig.3

Fig.4

Fig. 5

Fig.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1411333 A

**Littérature non-brevet citée dans la description**

- **J.R. ROMAN ; K.LEE ; S.A. PRAHL ; S.L. JACQUES.** Design, testing and clinical studies of a handheld polarized light camera. *Journal of Biomedical Optics,* 2004, vol. 9, 1305
- **COMPAIN, B. DRÉVILLON.** *Review of Scientific Instruments,* 1998, vol. 69, 1574
- **J.S. TYO.** *Optics Letters,* 2000, vol. 25, 1198
- **A. CHIPMAN.** Interpretation of Mueller matrices based on polar decomposition. *J. Opt. Soc. Am. A.,* 1996, vol. 13 (5), 1106